# EUROPÄISCHE PATENTANMELDUNG

(11) **EP 1 039 296 A1**
(43) Veröffentlichungstag der Anmeldung: **27.09.2000**
(21) Anmeldenummer: 99105695.3
(22) Anmeldetag: 19.03.1999
(51) Int. Cl.: G01N 33/49

(54) **System zur Gerinnungsmessung von Körperflüssigkeiten**

(71) Anmelder: Behnk, Holger, D-22417 Hamburg (DE)
(72) Erfinder: Behnk, Holger, D-22417 Hamburg (DE)
(74) Vertreter: Glawe, Delfs, Moll & Partner

(57) **Zusammenfassung**

Das System zur Gerinnungsmessung von Körperflüssigkeiten mit einer Küvette zum Aufnehmen einer Flüssigkeitsprobe und einer Meßverrichtung (10), die eine Einrichtung (13) zum Bewegen wenigstens eines Teils der Flüssigkeitsprobe und eine Einrichtung (15,16) zum Bestimmen des Gerinnungszeitpunkts mit einer Lichtquelle (16) und einem lichtempfindlichen Sensor (15) aufweist, zeichnet sich dadurch aus, daß die Küvette mehrere nebeneinander in einer Trennwand (4) zwischen zwei Ausnehmungen (2,3) angeordnete Durchgangsbohrungen (5) zum Aufnehmen der Flüssigkeitsprobe aufweist und daß die Einrichtung zum Bewegen ein Schwingungsgeber (13) für Druckwellen ist.

## Beschreibung

Die Erfindung betrifft ein System zur Gerinnungsmessung von Körperflüssigkeiten mit einer Küvette zum Aufnehmen einer Flüssigkeitsprobe und einer Meßvorrichtung, die eine Einrichtung zum Bewegen wenigstens eines Teils der Flüssigkeitsprobe und eine Einrichtung zum Bestimmen des Gerinnungszeitspunktes mit einer Lichtquelle und einem lichtempfindlichen Sensor aufweist.

Gerinnungsmessungen von Körperflüssigkeiten, insbesondere von Blut, müssen häufig aus verschiedenen medizinischen Gründen vorgenommen werden. Zu diesem Zweck sind spezielle Meßküvetten bekannt, die mit einem Analysegerät zusammenwirken, das in einem Labor die aufeinanderfolgende Messung einer großen Zahl von Proben gestattet (EP 0 369 168 B1). Mit diesem bekannten System kann das Gerinnungsverhalten von Proben untersucht werden, die an das Labor eingeschickt werden. In vielen Fällen ist aber eine Messung außerhalb des Labors erforderlich. Z. B. muß die Gerinnungsfähigkeit des Blutes bei Behandlung nach einem Herzinfarkt gemessen werden, wobei es wichtig ist, das Resultat sofort und nicht erst nach Stunden oder Tagen vom Labor zu erhalten. Eine schnelle Messung außerhalb des Labors ist auch bei Notfällen erforderlich. Vor der Notfalloperation muß zunächst die Gerinnungsfähigkeit des Blutes bestimmt werden, damit der Chirurg sicher ist, z. B. keinen Bluter vor sich zu haben. Auch hier ist die Untersuchung im Labor zu zeitaufwendig. Bei der Dialyse muß die Gerinnungsfähigkeit des Blutes praktisch auf Null herabgesetzt werden, damit die Filter nicht verstopfen. Der Patient kann erst nach Hause entlassen werden, wenn die Gerinnungsfähigkeit wieder einen gewissen Mindestwert angenommen hat. Auch hierfür ist eine Messung im Labor zumindest impraktikabel.

Es sind nun Systeme bekannt, bei denen die Messung auch außerhalb des Labors durchgeführt werden kann. Bei einem solchen vorbekannten System wird das Blut in eine Küvette eingebracht, die in eine Vorrichtung eingesetzt wird, mit der das Blut über ein Reagenz durch eine Kapillare hin- und hergepumpt wird (Prospekt "HEMOCHRON" der Firma International ITC TECHNIDYNE CORPORATION). Die Blutströmung wird dabei beobachtet. Wenn das Blut nicht mehr fließt, ist es geronnen.

Es ist außerdem bekannt, einen Blutstropfen auf ein kartenförmiges Element aufzubringen, von wo es dann durch Kapillaren zunächst durch einen Raum mit Reagenzien und dann durch eine längere Kapillare fließt. Der Weg, den das Blut dann noch zurücklegt, ist ein Maß für die Gerinnungsfähigkeit (Prospekt ,,Ciba Corning Gerinnungs-System der Firma Ciba Corning Diagnosics GmbH). Dieses Verfahren ist aber ungenau, da der noch vom Blut zurückgelegte Weg auch von der Viskosität abhängt und eine ungleichmäßige Mischung mit dem Reagenz stattfindet.

Bei einem System der eingangs genannten Art wird das Blut auf ein kartenförmiges Element gebracht und fließt dann durch eine Kapillare in einen Raum mit einem Reagenz, in dem sich auch ferromagnetische Teilchen befinden. Diese werden dann mit Hilfe eines Magneten bewegt, wodurch das Blut mit dem Reagenz gemischt wird. Mit optischen Mitteln wird dann festgestellt, wann sich die Bewegung in charakteristischer Weise verändert, was im Moment der Gerinnung der Fall ist (US 5,110,727). Die entsprechenden Karten, die in Deutschland von der Firma Boehringer Mannheim GmbH vertrieben werden, sind aber teuer in der Herstellung. Die Bedienung des entsprechenden Gerätes ist verhältnismäßig kompliziert und erfordert eine eintägige Schulung. So muß durch Betätigung einer "YES"-oder "NO-Taste" durch die Bedienungsperson eine Entscheidung getroffen werden, was ein solches Verfahren natürlich problematisch macht (Prospekt "CoaguCheck® der Firma Boehringer Mannheim GmbH).

Die Aufgabe der Erfindung besteht in der Schaffung eines Systems, mit dem einfach und zuverlässig die Gerinnungszeit von Körperflüssigkeiten gemessen werden kann, ohne daß eine komplizierte Behandlung erforderlich ist.

Die erfindungsgemäße Lösung besteht bei einem System der eingangs genannten Art darin, daß die Küvette eine oder mehrere nebeneinander in einer Trennwand zwischen zwei Ausnehmungen angeordnete Durchgangsbohrungen zum Aufnehmen der Flüssigkeitsprobe aufweist und daß die Einrichtung zum Bewegen ein Schwingungsgeber für Druckwellen ist.

Der Bluttropen oder sonstige Körperflüssigkeitstropfen wird im Bereich der Bohrung oder Bohrungen auf die Zwischenwand aufgebracht. Infolge Kapillarwirkung dringt dann die Flüssigkeit in die Bohrung oder Bohrungen ein. Anschließend werden mit einem Schwingungsgeber von oben oder von unten Luftdruckschwingungen erzeugt, die die Flüssigkeitssäulen in der Bohrung oder den Bohrungen membranartig zum Schwingen bringen. Die Flüssigkeit tritt dabei periodisch ein wenig aus den unteren Enden der Bohrung oder Bohrungen heraus und bewegt sich anschließend wieder hinein. Diese Schwingungen können mit Hilfe einer Lichtquelle und eines lichtempfindlichen Sensors für das zurückgestrahlte Licht detektiert werden. Diese Schwingungen werden selbstverständlich kleiner oder hören ganz auf, wenn die Gerinnung eingetreten ist. Der Beginn der Messung kann dabei automatisch festgestellt werden, nämlich in dem Moment, in dem die Schwingungen beginnen. Das Gerät braucht also nur einmal eingeschaltet zu werden; alles andere geschieht dann automatisch. Dies erleichtert die Bedienung selbstverständlich sehr.

Falls mehrere Bohrungen vorhanden sind, kann ein Mittelwert über die verschiedenen Bohrungen gebildet werden, da alle unteren Enden der Bohrungen mit einem einzigen Lichtfleck beleuchtet werden und daher das zurückgestrahlte Licht die Summe der Signale von allen Bohrungen darstellt. Dies erlaubt eine Mittelung und damit einen genaueren Wert, wobei kleine Toleranzunterschiede sich herausmitteln. In gewissen Fällen werden die Messungen mit Bohrungen durchgeführt, in denen sich kein Reagenz befindet. Wenn sich ein Reagenz dort befindet, so werden durch die Mittelwertbildung auch möglicherweise kleine Unterschiede in der Menge des Reagenz in den einzelnen Bohrungen ausgeglichen. Da die Bohrungen verhältnismäßig großen Durchmesser haben (als besonders vorteilhaft haben sich 0,8 bis 2 mm, insbesondere 1,0 bis 1,5 mm und ganz besonders ungefähr 1,2 mm erwiesen), kann in die Bohrungen bei Herstellung der Küvetten eingebrachtes Reagenz an den Wandungen in der Luft trocknen. Auf diese Weise bleibt das Reagenz stabil, selbst wenn es einige Zeit mit der Umgebungsluft in Berührung kommt.

Durch die gegenüber dem Stand der Technik größeren Durchmesser der Bohrungen bzw. Kapillaren spielen Viskositätsunterschiede nur eine geringe Rolle. Auch können diese Bohrungen nicht durch kleine Hautpartikel verstopft werden, die bei engen Kapillaren die Messung ungenau oder sogar unmöglich machen.

Wegen des verhältnismäßig großen Durchmessers der Kapillaren erhält man auch ein exakten Volumen und damit ein exaktes Verhältnis zwischen Probenflüssigkeit und Reagenz, was ebenfalls genauere Messungen ermöglicht.

Die erfindungsgemäße Küvette ist vorteilhafterweise aus einer Hülle und einem Einschub aufgebaut, so daß sie ohne große Schwierigkeiten und Kosten in großer Stückzahl geformt werden kann.

Zweckmäßigerweise sind die Öffnung oder Öffnungen der Küvette mit einer selbstklebenden Folie abgedeckt, die vor Beginn der Messung entfernt wird. Bei einer anderen vorteilhaften Ausführungsform sind die verhältnismäßig kleinen Küvetten in einer Blisterverpackung tablettenartig untergebracht, so daß sie bis zur Ingebrauchnahme ebenfalls hermetisch eingeschlossen, aber leicht aus der Verpackung zu entfernen sind.

Zweckmäßigerweise stehen die unteren Enden der Bohrungen von der Unterfläche der Trennwand vor. Es besteht so nicht die Gefahr, daß sich die unten aus den Bohrungen herauswölbenden Flüssigkeitstropfen miteinander vereinigen. Man beobachtet vielmehr dort separate Flüssigkeitstropfen.

Andererseits sind die Bohrungen an den oberen Enden von einer Randerhöhung umgeben, so daß hier ein gewisser Flüssigkeitstropfen über den Bohrungen stehen bleibt, der die vollständige Befüllung der Bohrungen sicherstellt. Die überschüssige Flüssigkeit über den Bohrungen hält die Probe auch auf mehr oder weniger konstanter Temperatur und schützt sie vor Austrocknung. Andererseits ist seitlich davon zweckmäßiger ein Aufnahmeraum vorgesehen, in den überflüssige Flüssigkeit abfließen kann.

Zweckmäßigerweise ist der Schwingungsgeber in einem Deckel der Meßvorrichtung angeordnet, wobei eine Luftdruckausgleichsöffnung im Deckel und/oder der Vorrichtung vorgesehen ist, damit beim Schließen des Deckels nicht eine Druckerhöhung auftritt, durch die die Flüssigkeit durch die Bohrungen hindurchgedrückt würde.

Diese Luftdruckausgleichsöffnung ist zweckmäßigerweise am Rand des Deckels vorgesehen. Bei dieser Ausführungsform dringen die Druckwellen durch die Einfüllöffnung für die Flüssigkeit in die Küvette ein.

Bei einer anderen Ausführungsform ist die Küvette mit einer unteren Eintrittsöffnung für Druckwellen versehen. Sie kann dann einfach auf die Meßvorrichtung aufgesetzt werden; ein Deckel ist nicht mehr erforderlich. Dadurch wird selbstverständlich die Messung schneller möglich.

Es wurde bereits erwähnt, daß die Bohrungen Durchmesser von 0,8 bis 2 mm haben. Als besonders vorteilhaft hat sich dabei eine Anzahl von sieben Bohrungen erwiesen, die auf engem Raum innerhalb eines Kreises angeordnet werden können. Die Länge der Bohrungen beträgt ungefähr 1 bis 3 mm, zweckmäßigerweise ungefähr 2 mm. Die Küvette kann daher verhältnismäßig klein gemacht werden und nimmt wenig Raum ein, was insbesondere die erwähnte tablettenartige Blisterverpackung ermöglicht.

Die Küvetten weisen zweckmäßigerweise eine Hülle aus lichtdurchlässigem Kunststoff, durch den hindurch dann auch die optische Messung durchgeführt werden kann. Der Hauptteil oder Einschub besteht dagegen vorteilhafterweise aus lichtundurchlässigem Kunststoff, da so die optische Messung leichter und zuverlässiger vorgenommen werden kann. Dabei kann auch vorgesehen sein, daß der Hauptteil je nach Reagenz unterschiedliche Farben hat.

Das System ist dabei zweckmäßigerweise so ausgebildet, daß jedem Satz von Küvetten eine Karte mit einem Mikrochip und/oder einem elektronischen Speicher beigefügt ist, in dem Information über das Reagenz, Eichwerte, Chargennummer und/oder Steuerinformation gespeichert ist. Diese Karte kann ungefähr die Form einer Telefonkarte haben.

Durch die Erfindung ist also eine einfache, zuverlässige und kostengünstige Messung der Gerinnungszeit möglich. Ein weiterer Vorteil besteht noch darin, daß im Gegensatz zu Systemen mit kartenförmigen Elementen keine Körperflüssigkeit, insbesondere kein Blut auf der Oberfläche der Karte bzw. Küvette zurückbleibt. Überschüssige Flüssigkeit wird vielmehr von der erfindungsgemäßen Küvette aufgenommen.

Die Erfindung wird im folgenden anhand von vorteilhaften Ausführungsformen unter Bezugnahme auf die beigefügten Zeichnungen bspw. beschrieben. Es zeigen in schematischer Darstellung:
- Fig. 1: den Hauptteil einer erfindungsgemäßen Küvette von unten;
- Fig. 2: den Hauptteil von Fig. 1 von der Seite in einem Schnitt entlang der Linie A-A;
- Fig. 3: den Hauptteil der Fig. 1 und 2 von oben;
- Fig. 4: einen Hauptteil einer Küvette, der gerade in eine entsprechende Umhüllung eingeschoben wird;
- Fig. 5: die Küvette der Fig. 4 von oben nach völligem Einschieben des Hauptteils;
- Fig. 6: eine Ausführungsform der Küvette in einer ersten Meßvorrichtung;
- Fig. 7: eine andere Ausführungsform der Küvette in einer anderen Meßvorrichtung;
- Fig. 8: mehrere Küvetten in einer Blisterverpackung von unten gesehen;
- Fig. 9: die Blisterverpackung mit Küvetten der Fig. 8 in einem Schnitt von der Seite;
- Fig. 10: die Oberseite der Blisterverpackung mit den Küvetten; und
- Fig. 11: eine Chipkarte, auf der die Daten der Reagenzien und der Küvetten gespeichert sind.

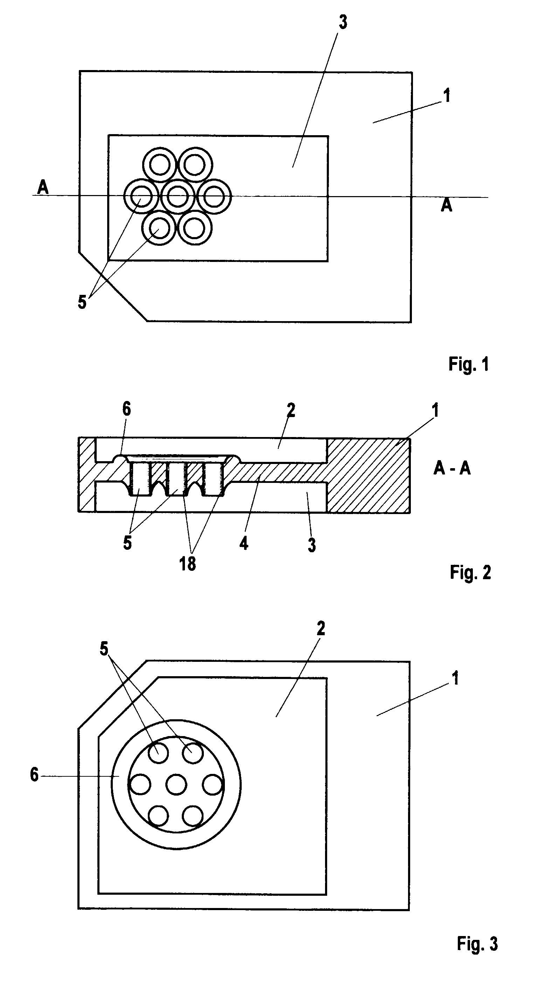

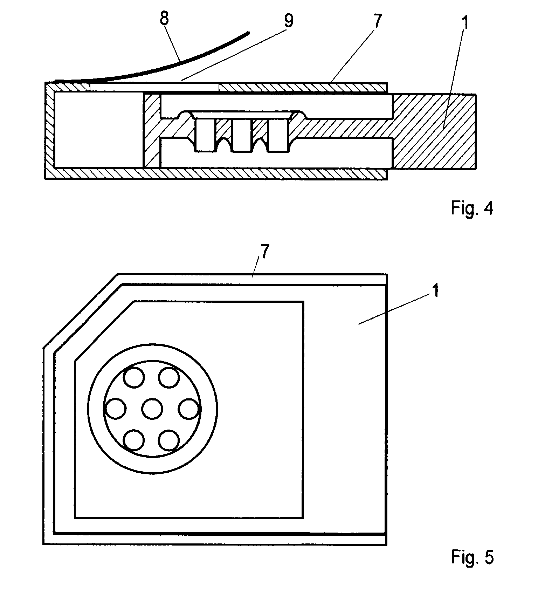

In den Fig. 1 bis 3 ist der Hauptteil 1 einer Küvette von unten, im Schnitt und von oben gezeigt. Der Hauptteil weist eine obere Ausnehmung 2 und eine untere Ausnehmung 3 auf, die durch eine Zwischenwand 4 getrennt sind. In dieser befinden sich Bohrungen 5, die nach unten vorstehen, wie dies am besten in Fig. 2 ersichtlich ist. Um die Bohrungen 5 herum ist ein Ringwulst 6 angeordnet, der es ermöglicht, daß ein Flüssigkeitstropfen oben auf den Bohrungen 5 stehenbleibt. Überflüssige Flüssigkeit kann dabei in den Raum 2 abfließen. Dies kann insbesondere durch Kapillarwirkung geschehen, wenn der Hauptteil 1 in eine Umhüllung 7 eingeschoben ist, die Fig. 4 gezeigt ist. Bei der Ausführungsform der Fig. 4 ist noch eine selbstklebende Verschlußfolie 8 gezeigt, die erst vor Ingebrauchnahme der Küvette entfernt wird. Diese verschließt eine obere Öffnung 9 in der Umhüllung 7, durch die Flüssigkeit eingefüllt wird. Die Umhüllung 7 besteht aus lichtdurchlässigem Kunststoff, der Hauptteil 1 ist vorteilhafterweise lichtundurchlässig. Bei 18 ist ein Reagenz angedeutet, daß sich auf den Wänden der Bohrungen befindet.

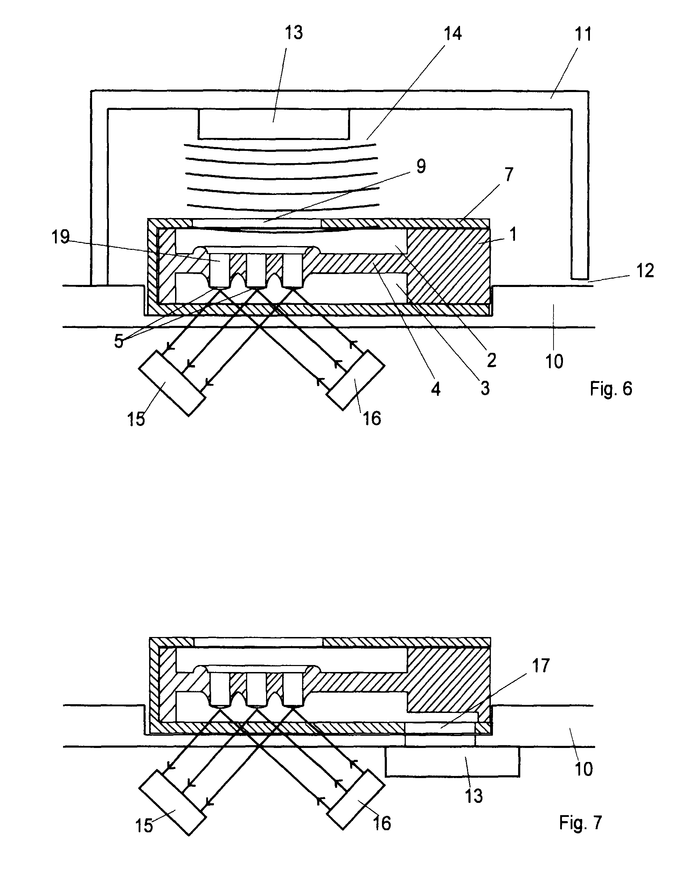

In Fig. 6 ist gezeigt, daß die Küvette der Fig. 1 bis 5 in eine Meßvorrichtung 10 eingesetzt ist, nachdem Flüssigkeit 19 oben auf die Bohrungen 5 aufgebracht worden ist, die unten an den Enden der Bohrungen 5 kleine Aufwölbungen bildet. Es wird dann ein Deckel 11 geschlossen, wobei eine Luftausgleichsöffnung 12 am Rand des Deckels 11 dafür sorgt, daß nicht ein Druckstoß entsteht, durch den die Flüssigkeit durch die Bohrungen 5 hindurch und aus diesen herausgedrückt würde. Anschließend wird dann ein Schwingungsgeber 13 eingeschaltet, durch den Luftdruckschwankungen erzeugt werden, die bei 14 angedeutet sind, und die Flüssigkeitssäulen in den Bohrungen 5 zum Schwingen bringen. Diese Schwingungen werden durch einen Detektor 15 detektiert, der zurückgestrahltes Licht von den unteren Enden der Bohrungen 5 detektiert. Zu diesem Zweck werden die unteren Enden der Bohrungen mit einem Lichtfleck durch eine Lichtquelle 16 beleuchtet. Durch die Schwingungen wird die Flüssigkeit in engem Kontakt mit dem Reagenz 18 gebracht, und es wird eine Durchmischung erreicht. Tritt Gerinnung ein, so hören die Schwingungen auf, wodurch der Gerinnungszeitpunkt festgelegt ist.

Bei der Ausführungsform der Fig. 7 ist in der Meßvorrichtung 10 der Schwingungsgeber 13 unten in der Meßvorrichtung 10 unterhalb der Meßküvette angeordnet. Damit die Schwingungen in die Küvette eindringen können, ist unten noch eine weiter Öffnung 17 bei dieser Ausführungsform vorgesehen. Die Küvette besteht aus durchsichtigem Kunststoff, wie dies bereits erwähnt wurde, so daß Licht von der Lichtquelle 16 ungehindert zum Sensor 15 gelangen kann.

Es sollte noch erwähnt werden, daß die Räume 2 und 3 verschiedenen Zwecken dienen. Der Raum 2 dient zur Aufnahme überschüssigen Blutes. Der Raum 3 ermöglicht bei der Ausführungsform, die unten geschlossen ist, überhaupt erst das Befüllen der Bohrungen mit Flüssigkeit, da dieser Raum einen Luftausgleichsraum bildet. Die Räume 2 und 3 bilden weiterhin Resonanzräume für die Schwingungen der Schwingungsgeber.

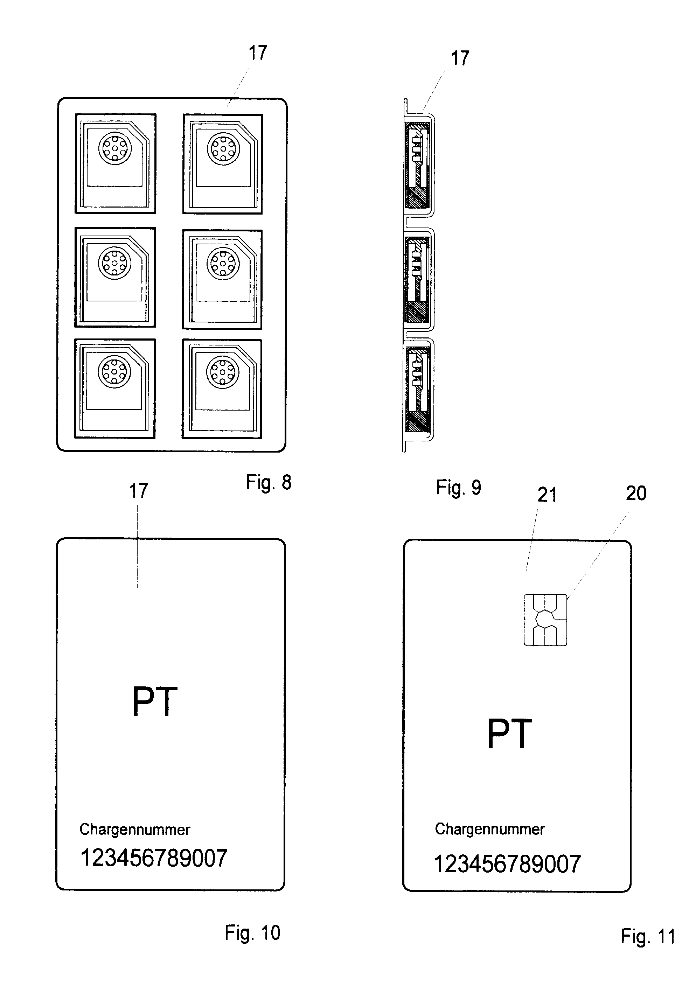

In Fig. 8 und 9 ist gezeigt, wie mehrere Küvetten in einer Blisterverpackung 17 angeordnet sind, wie sie für Tabletten bekannt ist. Die in Fig. 10 gezeigte Oberseite der Blisterverpackung trägt dabei eine Beschriftung mit insbesondere der Chargennummer. Diese Chargennummer befindet sich auch auf einer Chipkarte 21, die zusammen mit den Küvetten geliefert wird. Diese Chipkarte wird in die Vorrichtung 10 eingeschoben, wodurch diese eingeschaltet wird. Die Chipkarte enthält dabei die notwendigen Daten, Steueranweisungen usw., die für diese speziellen Küvetten maßgeblich sind.

## Patentansprüche

1. System zur Gerinnungsmessung von Körperflüssigkeiten mit einer Küvette zum Aufnehmen einer Flüssigkeitsprobe und einer Meßvorrichtung (10), die eine Einrichtung (13) zum Bewegen wenigstens eines Teils der Flüssigkeitsprobe und eine Einrichtung (15,16) zum Bestimmen des Gerinnungszeitpunkts mit einer Lichtquelle (16) und einem lichtempfindlichen Sensor (15) aufweist, dadurch gekennzeichnet, daß die Küvette eine oder mehrere nebeneinander in einer Trennwand (4) zwischen zwei Ausnehmungen (2,3) angeordnete Durchgangsbohrungen zum Aufnehmen der Flüssigkeitsprobe aufweist und daß die Einrichtung zum Bewegen ein Schwingungsgeber (13) für Druckwellen ist.

2. System nach Anspruch 1, dadurch gekennzeichnet, daß die Küvette aus einer Hülle (7) und einem Einschub (1) aufgebaut ist.

3. System nach Anspruch 1 oder 2, dadurch gekennzeichnet, daß die Öffnung oder Öffnungen (9,17) der Küvette mit einer selbstklebenden Folie (8) abgedeckt sind.

4. System nach einem der Ansprüche 1 bis 3, dadurch gekennzeichnet, daß die unteren Enden der Bohrungen (5) von der Unterfläche der Trennwand (4) vorstehen.

5. System nach einem der Ansprüche 1 bis 4, dadurch gekennzeichnet, daß der Schwingungsgeber (13) in einem Deckel (11) der Meßvorrichtung (10) angeordnet ist und daß eine Luftdruckausgleichsöffnung (12) im Deckel (11) und/oder der Vorrichtung (10) vorgesehen ist.

6. System nach Anspruch 5, dadurch gekennzeichnet, daß die Luftdruckausgleichsöffnung (12) am Rand des Deckels (11) vorgesehen ist.

7. System nach einem der Ansprüche 1 bis 4, dadurch gekennzeichnet, daß die Küvette mit einer unteren Eintrittsöffnung (17) für Druckwellen versehen ist.

8. System nach einem der Ansprüche 1 bis 7, dadurch gekennzeichnet, daß die Bohrungen (5) an ihren oberen Enden von einer Randerhöhung (6) umgeben sind.

9. System nach einem der Ansprüche 1 bis 8, dadurch gekennzeichnet, daß neben den oberen Enden der Bohrungen (5) ein Aufnahmeraum (2) für überschüssige Flüssigkeit vorgesehen ist.

10. System nach einem der Ansprüche 1 bis 9, dadurch gekennzeichnet, daß die Wandungen der Bohrungen (5) mit einem luftgetrockneten Reagenz (18) bedeckt sind.

11. System nach einem der Ansprüche 1 bis 9, dadurch gekennzeichnet, daß die Bohrungen (5) einen Durchmesser von ungefähr 0,8 bis 2 mm, vorzugsweise ungefähr 1,0 bis 1,5 mm, insbesondere ungefähr 1,2 mm haben.

12. System nach einem der Ansprüche 1 bis 11, dadurch gekennzeichnet, daß die Küvetten in einer Blisterverpackung (17) tablettenartig verpackt sind.

13. Systems nach einem der Ansprüche 1 bis 12, dadurch gekennzeichnet, daß der Beginn des Meßintervalls durch den lichtempfindlichen Sensor (15) bestimmt wird.

14. System nach einem der Ansprüche 1 bis 13, dadurch gekennzeichnet, daß die Hülle (7) der Küvette aus lichtdurchlässigem und der Einschub (1) aus lichtundurchlässigem Kunststoff besteht.

15. System nach einem der Ansprüche 1 bis 14, dadurch gekennzeichnet, daß jeder Satz von Küvetten eine Karte (21) mit einem Mikrochip und/oder elektronischem Speicher (20) aufweist, in dem Information über das Reagenz, Eichwerte, Chargennummer und/oder Steuerinformation gespeichert ist.
